# EUROPEAN PATENT SPECIFICATION

(11) **EP 2 016 185 B1**
(45) Date of publication and mention of the grant of the patent: **19.11.2014**
(21) Application number: 07760491.6
(22) Date of filing: 11.04.2007
(51) Int. Cl.: C12P 19/34

(54) **DETECTION OF INFLUENZA A VIRUS**
NACHWEIS VON INFLUENZA-A-VIRUS
DETECTION DE VIRUS DE LA GRIPPE A

(30) Priority: 11.04.2006 US 401648; 25.07.2006 US 459789
(43) Date of publication of application: 21.01.2009
(73) Proprietor: MAYO FOUNDATION FOR MEDICAL EDUCATION AND RESEARCH, Rochester, MN 55905 (US)
(72) Inventor: ESPY, Mark J., Rochester, Minnesota 55906 (US); SMITH, Thomas F., Rochester, Minnesota 55902 (US)
(74) Representative: Bösl, Raphael Konrad
(86) International application number: PCT/US2007/066440
(87) International publication number: WO 2007/121247

(56) References cited:
- WO-A1-2006/102695
- WO-A2-2004/057021
- PODDAR S: "Detection of type and subtypes of influenza virus by hybrid formation of FRET probe with amplified target DNA and melting temperature analysis" JOURNAL OF VIROLOGICAL METHODS, vol. 108, no. 2, March 2003 (2003-03), pages 157-163, XP002598807 ISSN: 0166-0934
- ZITTERKOPF N L ET AL: "Relevance of influenza a virus detection by PCR, shell vial assay, and tube cell culture to rapid reporting procedures." JOURNAL OF CLINICAL MICROBIOLOGY SEP 2006 LNKD- PUBMED:16954274, vol. 44, no. 9, September 2006 (2006-09), pages 3366-3367, XP002598808 ISSN: 0095-1137
- FOUCHIER R A M ET AL: "Detection of influenza A viruses from different species by PCR amplification of conserved sequences in the matrix gene" JOURNAL OF CLINICAL MICROBIOLOGY, AMERICAN SOCIETY FOR MICROBIOLOGY, WASHINGTON, DC, US, vol. 38, no. 11, 1 November 2000 (2000-11-01), pages 4096-4101, XP002555829 ISSN: 0095-1137
- LIND ET AL.: 'Combining Sequence-specific probes and DNA binding dyes in real-time PCR for specific nucleic acid quantification and melting curve analysis' BIOTECHNIQUES vol. 40, March 2006, pages 315 - 319, XP008131069
- HALL ET AL.: 'Variations in nucleotide sequences coding for the N-terminalregions of the matrix and nonstructural proteins of influenza A viruses' JOURNAL OF VIROLOGY vol. 38, no. 1, April 1981, pages 1 - 7, XP002442449
- SMITH ET AL.: 'Rapid detection of Influenza A and B viruses in clinical specimens by Light Cycler real time RT-PCR' JOURNAL OF CLINICAL VIROLOGY vol. 28, 2003, pages 51 - 58, XP008131019

### SEQUENCE LISTING

<110> Mayo Foundation for Medical Education and Research
<120> Detection of Influenza A Virus
<130> 20014/019WO1
<150> 11/459,789 <151> 2006-07-25
<140> 11/401,648 <141> 2006-04-11
<160> 4
<170> FastSEQ for Windows Version 4.0
<210> 1
   <211> 25
   <212> DNA
   <213> Artificial Sequence
<220>
   <223> oligonucleotide
<400> 1
   taaccgaggt cgaaacgtat gttct 25
<210> 2
   <211> 22
   <212> DNA
   <213> Artificial Sequence
<220>
   <223> oligonucleotide
<400> 2
   ggcattttgg acaaagcgtc ta 22
<210> 3
   <211> 27
   <212> DNA
   <213> Artificial Sequence
<220>
   <223> oligonucleotide
<400> 3
   cgaaatcgcg cagagacttg aagatgt 27
<210> 4
   <211> 28
   <212> DNA
   <213> Artificial Sequence
<220>
   <223> oligonucleotide
<400> 4
   ttgctgggaa aaacacagat cttgaggc 28

## Description

### TECHNICAL FIELD

This invention relates to viral diagnostics, and more particularly to the detection of influenza A virus.

### BACKGROUND

Influenza virus infects 5-20% of the population and results in 30,000-50,000 deaths each year in the U.S. Although the influenza vaccine is the primary method of infection prevention, four antiviral drugs are also available in the U.S.: amantadine, rimantadine, oseltamivir and zanamivir. As of December 2005, only oseltamivir (TAMIFLU®) is recommended for treatment of influenza A due to the increasing resistance of the virus to amantadine and rimantidine resulting from an amino acid substitution in the M2 protein of the virus.

Rapid and accurate laboratory diagnosis of influenza is crucial for initial detection, successful outbreak control within hospitals and the community, and for directing treatment. Importantly, there is a direct relationship between early administration of a drug and clinical response of the patient to the anti-influenza virus treatment. Oseltamivir, if given within the first 12 hours after the onset of fever, has been shown to reduce symptoms by about 72 hours. If given less than 48 hours post-symptom onset, illness has been reported to be reduced by 1-2 days. Importantly, the symptoms of influenza are difficult to distinguish from other viral and bacterial respiratory illnesses. Therefore, rapid identification of influenza virus infections provides the opportunity to initiate directed early antiviral treatment, and immediate reporting can prevent the unwarranted use of antibiotics. Further, accurate assessment of drug efficiency relies on the availability of accurate, rapid, sensitive, and specific test methods in the laboratory to detect influenza virus infections.

### SUMMARY

Described herein are methods of identifying influenza A virus nucleic acid by real-time polymerase chain reaction (PCR) in a biological sample. Primers and probes for detecting influenza A are described herein, as are kits containing such primers and probes. Methods of the invention can be used to rapidly identify influenza A nucleic acid from specimens for diagnosis of influenza A infection. Using specific primers and probes, the methods include amplifying and monitoring the development of specific amplification products using fluorescence resonance energy transfer (FRET).

There is provided a method for detecting the presence or absence of influenza A in a biological sample from an individual. The method to detect influenza A virus nucleic acid includes performing at least one cycling step, which includes an amplifying step and a hybridizing step. The amplifying step includes contacting the sample with a pair of influenza A primers to produce an influenza A amplification product if an influenza A nucleic acid molecule is present in the sample. The hybridizing step includes contacting the sample with a pair of influenza A probes. The members of the pair of influenza A probes hybridize within no more than five nucleotides of each other. A first influenza A probe of the pair of influenza A probes is labeled with a donor fluorescent moiety and a second influenza A probe of the pair of influenza A probes is labeled with a corresponding acceptor fluorescent moiety. The method further includes detecting the presence or absence of FRET between the donor fluorescent moiety of the first influenza A probe and the acceptor fluorescent moiety of the second influenza A probe. The presence of FRET is indicative of the presence of influenza A in the sample, while the absence of FRET is indicative of the absence of influenza A in the sample.

The pair of influenza A primers includes a first influenza A primer and a second influenza A primer. The first influenza A primer includes the sequence 5'-TAA CCG AGG TCG AAA CGT ATG TTC T-3' (SEQ ID NO:1) and the second influenza A primer includes the sequence 5'-GGC ATT TTG GAC AAA GCG TCT A-3' (SEQ ID NO:2). Moreover, the first influenza A probe includes the sequence 5'-CGA AAT CGC GCA GAG ACT TGA AGA TGT-3' (SEQ ID NO:3) and the second influenza A probe includes the sequence 5'-TTG CTG GGA AAA ACA CAG ATC TTG AGG C-3' (SEQ ID NO:4).

One of the influenza A primers can be labeled with a fluorescent moiety (either a donor or acceptor, as appropriate) and can take the place of one of the influenza A probes.

The members of the pair of influenza A probes can hybridize within no more than two nucleotides of each other, or can hybridize within no more than one nucleotide of each other. A representative donor fluorescent moiety is fluorescein, and corresponding acceptor fluorescent moieties include LC-Red 640, LC-Red 705, Cy5, and Cy5.5. Additional corresponding donor and acceptor fluorescent moieties are known in the art.

In one aspect, the detecting step includes exciting the sample at a wavelength absorbed by the donor fluorescent moiety and visualizing and/or measuring the wavelength emitted by the acceptor fluorescent moiety (*i.e.,* visualizing and/or measuring FRET). In another aspect, the detecting step includes quantitating the FRET. In yet another aspect, the detecting step can be performed after each cycling step (*e.g.,* in real-time).

Generally, the presence of FRET within 45 cycles (*e.g.,* 40, 35, 30, 25, or 20 cycles) indicates the presence of an influenza A infection in the individual. In addition, determining the melting temperature between one or both of the influenza A probe(s) and the influenza A amplification product can confirm the presence or absence of the influenza A.

Representative biological samples from the respiratory tract include throat swabs, throat washings, nasal swabs, and specimens from the lower respiratory tract. A biological sample also can include saliva as described in U.S. Patent No. 6,811,971.

The above-described methods can further include preventing amplification of a contaminant nucleic acid. Preventing amplification can include performing the amplifying step in the presence of uracil and treating the sample with uracil-DNA glycosylase prior to amplifying.

In addition, the cycling step can be performed on a control sample. A control sample can include the same portion of the influenza A nucleic acid molecule. Alternatively, a control sample can include a nucleic acid molecule other than an influenza A nucleic acid molecule. Cycling steps can be performed on such a control sample using a pair of control primers and a pair of control probes. The control primers and probes are other than influenza A primers and probes. One or more amplifying steps produces a control amplification product. Each of the control probes hybridizes to the control amplification product.

When influenza A is detected in a biological sample using the methods disclosed herein, the patient from which that biological sample was obtained can be provided with immediate treatment. Treatment, described herein, can include, for example, without limitation, administration of a neuraminidase inhibitor (e.g., Tamiflu®), quarantine, or both.

There are provided articles of manufacture, or kits. Kits may include a pair of influenza A primers, and a pair of influenza A probes, and a donor and corresponding acceptor fluorescent moieties as defined in the claims. The first influenza A primer provided in a kit described herein comprises the sequence 5'-TAA CCG AGG TCG AAA CGT CTG TTC T-3' (SEQ ID NO:1) and the second influenza A primer in a kit comprises the sequence 5'-GGC ATT TTG GAC AAA GCG TCT A-3' (SEQ ID NO:2). The first influenza A probe provided in a kit of the invention comprises the sequence 5'-CGA AAT CGC GCA GAG ACT TGA AGA TGT-3' (SEQ ID NO:3) and the second influenza A probe in a kit comprises the sequence 5'-TTG CTG GGA AAA ACA CAG ATC TTG AGG C-3' (SEQ ID NO:4).

Articles of manufacture or kits can include fluorophoric moieties for labeling the probes or probes already labeled with donor and corresponding acceptor fluorescent moieties. A kit can also include a package insert having instructions thereon for using the primers, probes, and fluorophoric moieties to detect the presence or absence of influenza A in a sample.

Also described herein is a method for detecting the presence or absence of influenza A in a biological sample from an individual. Such a method includes performing at least one cycling step. A cycling step can include an amplifying step and a hybridizing step. Generally, an amplifying step includes contacting the sample with a pair of influenza A primers to produce an influenza A amplification product if an influenza A nucleic acid molecule is present in the sample. Generally, a hybridizing step includes contacting the sample with an influenza A probe. Such an influenza A probe is usually labeled with a donor fluorescent moiety and a corresponding acceptor fluorescent moiety. The method further includes detecting the presence or absence of fluorescence resonance energy transfer (FRET) between the donor fluorescent moiety and the acceptor fluorescent moiety of the influenza A probe. The presence or absence of fluorescence is indicative of the presence or absence of influenza A in said sample.

Further described herein, amplification can employ a polymerase enzyme having 5' to 3' exonuclease activity. Thus, the first and second fluorescent moieties would be within no more than 5 nucleotides (e.g., 4, 3, 2, or 1 nucleotide) of each other along the length of the probe. Further described herein, the influenza A probe includes a nucleic acid sequence that permits secondary structure formation. Such secondary structure formation generally results in spatial proximity between the first and second fluorescent moiety. According to this method, the second fluorescent moiety on a probe can be a quencher.

Further described herein, there is provided a method for detecting the presence or absence of influenza A in a biological sample from an individual. Such a method includes performing at least one cycling step. A cycling step can include an amplifying step and a dye-binding step. An amplifying step generally includes contacting the sample with a pair of influenza A primers to produce an influenza A amplification product if an influenza A nucleic acid molecule is present in the sample. A dye-binding step generally includes contacting the influenza A amplification product with a double-stranded DNA binding dye. The method further includes detecting the presence or absence of binding of the double-stranded DNA binding dye into the amplification product. Further described herein, the presence of binding is typically indicative of the presence of influenza A in the sample, and the absence of binding is typically indicative of the absence of influenza A in the sample. Such a method can further include the steps of determining the melting temperature between the influenza A amplification product and the double-stranded DNA binding dye. Generally, the melting temperature confirms the presence or absence of influenza A. Representative double-stranded DNA binding dyes include SYBRGREEN I®, SYBRGOLD®, and ethidium bromide.

The methods according to the invention can be used to determine whether or not an individual is in need of treatment for influenza A. Described herein, treatment for influenza A can include, for example, administration of a neuraminidase inhibitor (e.g., Tamiflu®) to the individual. The articles of manufacture described herein can be used to determine whether or not an individual is in need of treatment for influenza A. Further, the methods and/or the articles of manufacture described herein can be used to monitor an individual for the effectiveness of a treatment for influenza A as well as in epidemiology to monitor the transmission and progression of influenza A from individuals to individuals in a population (e.g., a village, a city, or a country) and/or populations to populations.

The methods and/or the articles of manufacture (e.g., kits) disclosed herein can be used to determine whether or not a patient is in need of treatment for influenza A.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting. In case of conflict, the present specification, including definitions, will control.

The details of one or more embodiments of the invention are set forth in the accompanying drawings and the description below. Other features, objects, and advantages of the invention will be apparent from the drawings and detailed description, and from the claims.

### DETAILED DESCRIPTION

A real-time assay for detecting influenza A in a biological sample that is more sensitive and specific than existing assays is described herein. Primers and probes for detecting influenza A infections and articles of manufacture containing such primers and probes are provided herein. The increased sensitivity of real-time PCR for detection of influenza A compared to other methods, as well as the improved features of real-time PCR including sample containment and real-time detection of the amplified product, make feasible the implementation of this technology for routine diagnosis of influenza A infections in the clinical laboratory.

### Influenza A nucleic acids and oligonucleotides

Described herein are methods to detect influenza A by amplifying, for example, a portion of an influenza A nucleic acid. Nucleic acid sequences from influenza A are available. See, for example, the Influenza Sequence Database (ISD) (flu.lanl.gov on the World Wide Web, described in Macken et al., 2001, "The value of a database in surveillance and vaccine selection" in Options for the Control of Influenza IV. A.D.M.E., Osterhaus & Hampson (Eds.), Elsevier Science, Amsterdam, pp. 103-106) and the Microbial Sequencing Center (MSC) at The Institute for Genomic Research (TIGR) (tigr.org/msc/infl_a_virus.shtml on the World Wide Web).

Specifically, primers and probes to amplify and detect influenza A nucleic acid molecules are described herein. Influenza A nucleic acids other than those exemplified herein also can be used to detect influenza A in a sample. Influenza A nucleic acids other than those exemplified herein (e.g., functional variants) can be evaluated (e.g., for specificity and/or sensitivity) by those of skill in the art using routine methods such as, but not limited to, the methods exemplified herein. Representative functional variants, for example, include deletions of, insertions in, and/or substitutions in the nucleic acids disclosed herein.

Primers that amplify an influenza A nucleic acid molecule, *e.g*., influenza A can be designed using, for example, a computer program such as OLIGO (Molecular Biology Insights, Inc., Cascade, CO). Important features when designing oligonucleotides to be used as amplification primers include, but are not limited to, an appropriate size amplification product to facilitate detection (*e.g*., by electrophoresis), similar melting temperatures for the members of a pair of primers, and the length of each primer (*i.e*., the primers need to be long enough to anneal with sequence-specificity and to initiate synthesis but not so long that fidelity is reduced during oligonucleotide synthesis). Typically, oligonucleotide primers are 15 to 30 (e.g., 16, 18, 20, 21, 22, 23, 24, or 25) nucleotides in length.

Designing oligonucleotides to be used as hybridization probes can be performed in a manner similar to the design of primers, although the members of a pair of probes preferably anneal to an amplification product within no more than 5 nucleotides of each other on the same strand such that FRET can occur (*e.g*., within no more than 1, 2, 3, or 4 nucleotides of each other). This minimal degree of separation typically brings the respective fluorescent moieties into sufficient proximity such that FRET occurs. It is to be understood, however, that other separation distances (*e.g*., 6 or more nucleotides) are possible provided the fluorescent moieties are appropriately positioned relative to each other (for example, with a linker arm) such that FRET can occur. In addition, probes can be designed to hybridize to targets that contain a polymorphism or mutation, thereby allowing differential detection of influenza A strains based on either absolute hybridization of different pairs of probes corresponding to the particular influenza A strain to be distinguished or differential melting temperatures between, for example, members of a pair of probes and each amplification product corresponding to an influenza A strain to be distinguished. As with oligonucleotide primers, oligonucleotide probes usually have similar melting temperatures, and the length of each probe must be sufficient for sequence-specific hybridization to occur but not so long that fidelity is reduced during synthesis. Oligonucleotide probes are generally 15 to 30 (e.g., 16, 18, 20, 21, 22, 23, 24, or 25) nucleotides in length.

Constructs include vectors containing an influenza A nucleic acid molecule (e.g., SEQ ID NOs:1, 2, 3, or 4). Constructs can be used, for example, as control template nucleic acid molecules. Vectors suitable for use are commercially available and/or produced by recombinant nucleic acid technology methods routine in the art. Influenza A nucleic acid molecules can be obtained, for example, by chemical synthesis, direct cloning from influenza A, or by PCR amplification. An influenza A nucleic acid molecule or fragment thereof can be operably linked to a promoter or other regulatory element such as an enhancer sequence, a response element, or an inducible element that modulates expression of the influenza A nucleic acid molecule. As used herein, operably linking refers to connecting a promoter and/or other regulatory elements to an influenza A nucleic acid molecule in such a way as to permit and/or regulate expression of the influenza A nucleic acid molecule. A promoter that does not normally direct expression of influenza A can be used to direct transcription of an influenza A nucleic acid using, for example, a viral polymerase, a bacterial polymerase, or a eukaryotic RNA polymerase II. Alternatively, the influenza A native promoter can be used to direct transcription of an influenza A nucleic acid. In addition, operably linked can refer to an appropriate connection between an influenza A promoter or regulatory element and a heterologous coding sequence *(i.e.,* a non-influenza A coding sequence, for example, a reporter gene) in such a way as to permit expression of the heterologous coding sequence.

Constructs suitable for use in the methods described herein typically include, in addition to influenza A nucleic acid molecules (e.g., a nucleic acid molecule that contains one or more sequences of SEQ ID NOs:1, 2, 3, or 4), sequences encoding a selectable marker (*e.g.,* an antibiotic resistance gene) for selecting desired constructs and/or transformants, and an origin of replication. The choice of vector systems usually depends upon several factors, including, but not limited to, the choice of host cells, replication efficiency, selectability, inducibility, and the ease of recovery.

Constructs containing influenza A nucleic acid molecules can be propagated in a host cell. As used herein, the term host cell is meant to include prokaryotes and eukaryotes such as yeast, plant and animal cells. Prokaryotic hosts may include *E. coli, Salmonella typhimurium, Serratia marcescens* and *Bacillus subtilis.* Eukaryotic hosts include yeasts such as *S. cerevisiae, S. pombe, Pichia pastoris,* mammalian cells such as COS cells or Chinese hamster ovary (CHO) cells, insect cells, and plant cells such as *Arabidopsis thaliana* and *Nicotiana tabacum.* A construct can be introduced into a host cell using any of the techniques commonly known to those of ordinary skill in the art. For example, calcium phosphate precipitation, electroporation, heat shock, lipofection, microinjection, and viral-mediated nucleic acid transfer are common methods for introducing nucleic acids into host cells. In addition, naked DNA can be delivered directly to cells (see, *e.g.,* U.S. Patent Nos. 5,580,859 and 5,589,466).

### Polymerase chain reaction (PCR)

U.S. Patent Nos. 4,683,202, 4,683,195, 4,800,159, and 4,965,188 disclose conventional PCR techniques. PCR typically employs two oligonucleotide primers that bind to a selected nucleic acid template (e.g., DNA or RNA). Primers useful include oligonucleotides capable of acting as a point of initiation of nucleic acid synthesis within influenza A nucleic acid sequences (e.g., SEQ ID NO:1, 2, 3, or 4). A primer can be purified from a restriction digest by conventional methods, or it can be produced synthetically. The primer is preferably single-stranded for maximum efficiency in amplification, but the primer can be double-stranded. Double-stranded primers are first denatured, *i.e*., treated to separate the strands. One method of denaturing double stranded nucleic acids is by heating.

The term "thermostable polymerase" refers to a polymerase enzyme that is heat stable, *i.e*., the enzyme catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus flavus, T. ruber, T. thermophilus, T. aquaticus*, *T. lacteus, T. rubens, Bacillus stearothermophilus,* and *Methanothermus fervidus.* Nonetheless, polymerases that are not thermostable also can be employed in PCR assays provided the enzyme is replenished.

If the template nucleic acid is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (*e.g*., greater than 50%, 60%, 70%, 80%, 90% or 95% denatured). The heating conditions necessary for denaturing template nucleic acid will depend, *e.g.,* on the buffer salt concentration and the length and nucleotide composition of the nucleic acids being denatured, but typically range from about 90°C to about 105°C for a time depending on features of the reaction such as temperature and the nucleic acid length. Denaturation is typically performed for about 30 sec to 4 min (e.g., 1 min to 2 min 30 sec, or 1.5 min).

If the double-stranded template nucleic acid is denatured by heat, the reaction mixture is allowed to cool to a temperature that promotes annealing of each primer to its target sequence on the influenza A nucleic acid. The temperature for annealing is usually from about 35°C to about 65°C (e.g., about 40°C to about 60°C; about 45°C to about 50°C). Annealing times can be from about 10 secs to about 1 min (e.g., about 20 sec to about 50 sec; about 30 sec to about 40 sec). The reaction mixture is then adjusted to a temperature at which the activity of the polymerase is promoted or optimized, *i.e.,* a temperature sufficient for extension to occur from the annealed primer to generate products complementary to the template nucleic acid. The temperature should be sufficient to synthesize an extension product from each primer that is annealed to a nucleic acid template, but should not be so high as to denature an extension product from its complementary template (*e.g.*, the temperature for extension generally ranges from about 40° to 80°C (e.g., about 50°C to about 70°C; about 60°C). Extension times can be from about 10 secs to about 5 mins (e.g., about 30 sec to about 4 min; about 1 min to about 3 min; about 1 min 30 sec to about 2 min).

PCR assays can employ influenza A nucleic acid such as RNA or DNA (cDNA). The template nucleic acid need not be purified; it may be a minor fraction of a complex mixture, such as influenza A nucleic acid contained in human cells. Influenza A nucleic acids may be extracted from a biological sample by routine techniques such as those described in Diagnostic Molecular Microbiology: Principles and Applications (Persing et al. (eds), 1993, American Society for Microbiology, Washington D.C) or U.S. Patent No. 6,811,971. Nucleic acids can be obtained from any number of sources, such as plasmids, or natural sources including bacteria, yeast, viruses, organelles, or higher organisms such as plants or animals.

The oligonucleotide primers (e.g., SEQ ID NO:1 or 2) are combined with PCR reagents under reaction conditions that induce primer extension. For example, chain extension reactions generally include 50 mM KCl, 10 mM Tris-HCl (pH 8.3), 15 mM MgCl₂, 0.001% (w/v) gelatin, 0.5-1.0 µg denatured template DNA, 50 pmoles of each oligonucleotide primer, 2.5 U of Taq polymerase, and 10% DMSO). The reactions usually contain 150 to 320 µM each of dATP, dCTP, dTTP, dGTP, or one or more analogs thereof.

The newly synthesized strands form a double-stranded molecule that can be used in the succeeding steps of the reaction. The steps of strand separation, annealing, and elongation can be repeated as often as needed to produce the desired quantity of amplification products corresponding to the target influenza A nucleic acid molecule. The limiting factors in the reaction are the amounts of primers, thermostable enzyme, and nucleoside triphosphates present in the reaction. The cycling steps *(i.e.,* denaturation, annealing, and extension) are preferably repeated at least once. For use in detection, the number of cycling steps will depend, *e.g.,* on the nature of the sample. If the sample is a complex mixture of nucleic acids, more cycling steps will be required to amplify the target sequence sufficient for detection. Generally, the cycling steps are repeated at least about 20 times, but may be repeated as many as 40, 60, or even 100 times.

### Fluorescence resonance energy transfer (FRET)

FRET technology (see, for example, U.S. Patent Nos. 4,996,143, 5,565,322, 5,849,489, and 6,162,603) is based on a concept that when a donor and a corresponding acceptor fluorescent moiety are positioned within a certain distance of each other, energy transfer takes place between the two fluorescent moieties that can be visualized or otherwise detected and/or quantitated. Two oligonucleotide probes (e.g., SEQ ID NO:3 or 4), each containing a fluorescent moiety, can hybridize to an amplification product at particular positions determined by the complementarity of the oligonucleotide probes to the influenza A target nucleic acid sequence. Upon hybridization of the oligonucleotide probes to the amplification product nucleic acid at the appropriate positions, a FRET signal is generated. Hybridization temperatures can range from about 35°C to about 65°C (e.g., about 40°C to about 60°C; about 45°C to about 55°C; about 50°C) for about 10 sec to about 1 min (e.g., about 20 sec to about 50 sec; about 30 sec to about 40 sec).

Fluorescent analysis can be carried out using, for example, a photon counting epifluorescent microscope system (containing the appropriate dichroic mirror and filters for monitoring fluorescent emission at the particular range), a photon counting photomultiplier system or a fluorometer. Excitation to initiate energy transfer can be carried out with an argon ion laser, a high intensity mercury (Hg) arc lamp, a fiber optic light source, or other high intensity light source appropriately filtered for excitation in the desired range.

As used herein with respect to donor and corresponding acceptor fluorescent moieties "corresponding" refers to an acceptor fluorescent moiety having an emission spectrum that overlaps the excitation spectrum of the donor fluorescent moiety. The wavelength maximum of the emission spectrum of the acceptor fluorescent moiety should be at least 100 nm greater than the wavelength maximum of the excitation spectrum of the donor fluorescent moiety. Accordingly, efficient non-radiative energy transfer can be produced therebetween.

Fluorescent donor and corresponding acceptor moieties are generally chosen for (a) high efficiency Förster energy transfer; (b) a large final Stokes shift (>100 nm); (c) shift of the emission as far as possible into the red portion of the visible spectrum (>600 nm); and (d) shift of the emission to a higher wavelength than the Raman water fluorescent emission produced by excitation at the donor excitation wavelength. For example, a donor fluorescent moiety can be chosen that has its excitation maximum near a laser line (for example, Helium-Cadmium 442 nm or Argon 488 nm), a high extinction coefficient, a high quantum yield, and a good overlap of its fluorescent emission with the excitation spectrum of the corresponding acceptor fluorescent moiety. A corresponding acceptor fluorescent moiety can be chosen that has a high extinction coefficient, a high quantum yield, a good overlap of its excitation with the emission of the donor fluorescent moiety, and emission in the red part of the visible spectrum (>600 nm).

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothio-cyanatostilbene-2,2'-disulfonic acid, 7-diethylamino-3-(4'-isothiocyanatophenyl)-4-methylcoumarin, succinimidyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC™-Red 640, LC™-Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (*e.g*., Europium, or Terbium). Donor and acceptor fluorescent moieties can be obtained, for example, from Molecular Probes (Junction City, OR) or Sigma Chemical Co. (St. Louis, MO).

The donor and acceptor fluorescent moieties can be attached to the appropriate probe oligonucleotide via a linker arm. The length of each linker arm is important, as the linker arms will affect the distance between the donor and acceptor fluorescent moieties. The length of a linker arm for the purpose of the present invention is the distance in Angstroms (Å) from the nucleotide base to the fluorescent moiety. In general, a linker arm is from about 10 to about 25 Å (e.g., about 15Å to about 20Å). The linker arm may be of the kind described in WO 84/03285. WO 84/03285 also discloses methods for attaching linker arms to a particular nucleotide base, and also for attaching fluorescent moieties to a linker arm.

An acceptor fluorescent moiety such as an LC™-Red 640-NHS-ester can be combined with C6-Phosphoramidites (available from ABI (Foster City, CA) or Glen Research (Sterling, VA)) to produce, for example, LC™-Red 640-Phosphoramidite. Frequently used linkers to couple a donor fluorescent moiety such as fluorescein to an oligonucleotide include thiourea linkers (FITC-derived, for example, fluorescein-CPG's from Glen Research or ChemGene (Ashland, MA)), amide-linkers (fluorescein-NHS-ester-derived, such as fluorescein-CPG from BioGenex (San Ramon, CA)), or 3'-amino-CPG's that require coupling of a fluorescein-NHS-ester after oligonucleotide synthesis.

### Detection of Influenza A

Traditionally, laboratory methods have been used for diagnosis of influenza virus type A infections. One assay is the detection of virus-infected cells from the respiratory tract by direct immunofluorescence (DFA). The sensitivity of this labor-intensive DFA procedure, however, can be as low as 67% compared to cell cultures. In addition, the quality and interpretation of results can be difficult, subjective, and dependent on the quality of the specimens and microscopic equipment. In routine virology practice, DFA is not a stand-alone diagnostic technique for the diagnosis of influenza virus A infections.

As a substitute for conventional tube cell cultures, which rely on the recognition of CPE and/or hemadsorption to detect influenza viruses, R-Mix shell vial cell cultures have been developed that reduce the detection of influenza virus from several days to one or two days after receipt of the specimen into the laboratory. Reports expressed concern, however, that if one of two R-Mix shell vial cell cultures inoculated with a specimen from the respiratory tract stained positive with a pooled stain for respiratory viruses (immunofluorescence), the second R-Mix vial examined later did not always yield an identifiable virus. This presumably occurred because of a low titer of virus that could not be identified by staining the cells from the second R-Mix shell vial, which causes a delay in reporting results.

By using commercially available real-time PCR instrumentation (*e.g*., LIGHTCYCLER™, Roche Molecular Biochemicals, Indianapolis, IN), however, PCR amplification and detection of the amplification product can be combined in a single closed cuvette with dramatically reduced cycling time. Since detection occurs concurrently with amplification, the real-time PCR methods obviate the need for manipulation of the amplification product, and diminish the risk of cross-contamination between amplification products. Real-time PCR greatly reduces turn-around time and is an attractive alternative to conventional PCR techniques in the clinical laboratory.

The present invention provides methods for detecting the presence or absence of influenza A in a biological sample from an individual. Methods provided by the invention avoid problems of sample contamination, false negatives, and false positives. The methods can be used to determine whether or not a patient is in need of treatment for influenza A. If positive, the patient can be administered an appropriate medication (e.g., a neuraminidase inhibitor such as TAMIFLU®) in a timely manner. Prompt administration can reduce the amount of infection and the amount of symptoms. Prompt or even pre-screening individuals for influenza A can be used to identify carriers of the virus, which in turn can be used to reduce or eliminate the transmission of virus from carriers to non-carriers.

The methods include performing at least one cycling step that includes amplifying a portion of an influenza A nucleic acid molecule from a sample using a pair of influenza A primers. Each of the influenza A primers anneals to a target within or adjacent to an influenza A nucleic acid molecule such that at least a portion of each amplification product contains nucleic acid sequence corresponding to influenza A. More importantly, the amplification product should contain the nucleic acid sequences that are complementary to the influenza A probes. The influenza A amplification product is produced provided that influenza A nucleic acid is present. Each cycling step further includes contacting the sample with a pair of influenza A probes. According to the invention, one member of each pair of the influenza A probes is labeled with a donor fluorescent moiety and the other is labeled with a corresponding acceptor fluorescent moiety. The presence or absence of FRET between the donor fluorescent moiety of the first influenza A probe and the corresponding acceptor fluorescent moiety of the second influenza A probe is detected upon hybridization of the influenza A probes to the influenza A amplification product.

Each cycling step includes an amplification step and a hybridization step, and each cycling step is usually followed by a FRET detecting step. Multiple cycling steps are performed, preferably in a thermocycler. Methods of the invention can be performed using the influenza A primer and probe set to detect the presence of influenza A. Detection of FRET in the influenza A reaction indicates the presence of an influenza A.

As used herein, "amplifying" refers to the process of synthesizing nucleic acid molecules that are complementary to one or both strands of a template nucleic acid molecule (*e.g*., influenza A nucleic acid molecules). Amplifying a nucleic acid molecule typically includes denaturing the template nucleic acid, annealing primers to the template nucleic acid at a temperature that is below the melting temperatures of the primers, and enzymatically elongating from the primers to generate an amplification product. Amplification typically requires the presence of deoxyribonucleoside triphosphates, a DNA polymerase enzyme (*e.g*., PLATINUM^{®} Taq) and an appropriate buffer and/or co-factors for optimal activity of the polymerase enzyme (*e.g*., MgCl₂ and/or KCl).

If amplification of influenza A nucleic acid occurs and an amplification product is produced, the step of hybridizing results in a detectable signal based upon FRET between the members of the pair of probes. As used herein, "hybridizing" refers to the annealing of probes to an amplification product. Hybridization conditions typically include a temperature that is below the melting temperature of the probes but that avoids non-specific hybridization of the probes.

Generally, the presence of FRET indicates the presence of influenza A in the sample, and the absence of FRET indicates the absence of influenza A in the sample. Inadequate specimen collection, transportation delays, inappropriate transportation conditions, or use of certain collection swabs (e.g., calcium alginate swabs or aluminum shaft swabs) are all conditions that can affect the success and/or accuracy of a test result, however. Using the methods disclosed herein, detection of FRET within 45 cycling steps is indicative of an influenza A infection.

Methods of the invention also can be used for influenza A vaccine efficacy studies or epidemiology studies. For example, an influenza A vaccine can be detected using the methods of the invention during the time when virus is still present in an individual. For such vaccine efficacy studies, the methods of the invention can be used to determine, for example, the persistence of an attenuated strain of influenza A used in a vaccine, or can be performed in conjunction with an additional assay such as a serologic assay to monitor an individual's immune response to such a vaccine. In addition, methods of the invention can be used to distinguish one influenza A strain from another for epidemiology studies of, for example, the origin or severity of an outbreak of influenza A. In addition, monitoring how long influenza persists in an infected patient enables hospital infection control teams to limit and possibly reduce nosocomial transmission.

Representative biological samples that can be used in practicing the methods of the invention include, without limitation, throat swabs, throat washings, nasal washings, and specimens from the lower respiratory tract. U.S. Patent No. 6,811,971 describes the use of saliva to detect the presence or absence of influenza A or influenza B. Collection and storage methods of biological samples are known to those of skill in the art. Biological samples can be processed (*e.g*., by nucleic acid extraction methods and/or kits known in the art) to release influenza A nucleic acid or in some cases, the biological sample can be contacted directly with the PCR reaction components and the appropriate oligonucleotides.

Melting curve analysis is an additional step that can be included in a cycling profile. Melting curve analysis is based on the fact that DNA melts at a characteristic temperature called the melting temperature (Tm), which is defined as the temperature at which half of the DNA duplexes have separated into single strands. The melting temperature of a DNA depends primarily upon its nucleotide composition. Thus, DNA molecules rich in G and C nucleotides have a higher Tm than those having an abundance of A and T nucleotides. By detecting the temperature at which signal is lost, the melting temperature of probes can be determined. Similarly, by detecting the temperature at which signal is generated, the annealing temperature of probes can be determined. The melting temperature(s) of the influenza A probes from the influenza A amplification product can confirm the presence or absence of influenza A in the sample.

Within each thermocycler run, control samples are cycled as well. Positive control samples can amplify influenza A nucleic acid control template (e.g., other than influenza A) using, for example, control primers and control probes. Positive control samples can also amplify, for example, a plasmid construct containing influenza A nucleic acid molecules. Such a plasmid control can be amplified internally (*e.g*., within the sample) or in a separate sample run side-by-side with the patients' samples. Each thermocycler run should also include a negative control that, for example, lacks influenza A template DNA. Such controls are indicators of the success or failure of the amplification, hybridization and/or FRET reaction. Therefore, control reactions can readily determine, for example, the ability of primers to anneal with sequence-specificity and to initiate elongation, as well as the ability of probes to hybridize with sequence-specificity and for FRET to occur.

The methods described herein may include steps to avoid contamination. For example, an enzymatic method utilizing uracil-DNA glycosylase is described in U.S. Patent Nos. 5,035,996, 5,683,896 and 5,945,313 to reduce or eliminate contamination between one thermocycler run and the next. In addition, standard laboratory containment practices and procedures are desirable when performing methods of the invention. Containment practices and procedures include, but are not limited to, separate work areas for different steps of a method, containment hoods, barrier filter pipette tips and dedicated air displacement pipettes. Consistent containment practices and procedures by personnel are necessary for accuracy in a diagnostic laboratory handling clinical samples.

Conventional PCR methods in conjunction with FRET technology can be used to practice the methods of the invention. A LIGHTCYCLER™ instrument can be used. A detailed description of the LIGHTCYCLER™ System and real-time and on-line monitoring of PCR can be found at biochem.roche.com/lightcycler on the World Wide Web. The following patent applications describe real-time PCR as used in the LIGHTCYCLER™ technology: WO 97/46707, WO 97/46714 and WO 97/46712. The LIGHTCYCLER™ instrument is a rapid thermal cycler combined with a microvolume fluorometer utilizing high quality optics. This rapid thermocycling technique uses thin glass cuvettes as reaction vessels. Heating and cooling of the reaction chamber are controlled by alternating heated and ambient air. Due to the low mass of air and the high ratio of surface area to volume of the cuvettes, very rapid temperature exchange rates can be achieved within the LIGHTCYCLER™ thermal chamber. Addition of selected fluorescent dyes to the reaction components allows the PCR to be monitored in real time and on-line. Furthermore, the cuvettes serve as an optical element for signal collection (similar to glass fiber optics), concentrating the signal at the tip of the cuvette. The effect is efficient illumination and fluorescent monitoring of microvolume samples.

The LIGHTCYCLER™ carousel that houses the cuvettes can be removed from the instrument. Therefore, samples can be loaded outside of the instrument (in a PCR Clean Room, for example). In addition, this feature allows for the sample carousel to be easily cleaned and sterilized. The fluorometer, as part of the LIGHTCYCLER™ apparatus, houses the light source. The emitted light is filtered and focused by an epi-illumination lens onto the top of the cuvette. Fluorescent light emitted from the sample is then focused by the same lens, passed through a dichroic mirror, filtered appropriately, and focused onto data-collecting photohybrids. The optical unit currently available in the LIGHTCYCLER™ instrument (Roche Molecular Biochemicals, Catalog No. 2011 468) includes three band-pass filters (530 nm, 640 nm, and 710 nm), providing three-color detection and several fluorescence acquisition options. Data collection options include once per cycling step monitoring, fully continuous single-sample acquisition for melting curve analysis, continuous sampling (in which sampling frequency is dependent on sample number) and/or stepwise measurement of all samples after defined temperature interval.

The LIGHTCYCLER™ can be operated using a PC workstation and can utilize a Windows NT operating system. Signals from the samples are obtained as the machine positions the capillaries sequentially over the optical unit. The software can display the fluorescence signals in real-time immediately after each measurement. Fluorescent acquisition time is 10-100 milliseconds (msec). After each cycling step, a quantitative display of fluorescence vs. cycle number can be continually updated for all samples. The data generated can be stored for further analysis.

As an alternative to FRET, an amplification product can be detected using a double-stranded DNA binding dye such as a fluorescent DNA binding dye (*e.g*., SYBRGREEN I^{®} or SYBRGOLD^{®} (Molecular Probes)). Upon interaction with the double-stranded nucleic acid, such fluorescent DNA binding dyes emit a fluorescence signal after excitation with light at a suitable wavelength. A double-stranded DNA binding dye such as a nucleic acid intercalating dye also can be used. When double-stranded DNA binding dyes are used, a melting curve analysis is usually performed for confirmation of the presence of the amplification product.

As described herein, amplification products also can be detected using labeled hybridization probes that take advantage of FRET technology. A common format of FRET technology utilizes two hybridization probes. Each probe can be labeled with a different fluorescent moiety and are generally designed to hybridize in close proximity to each other in a target DNA molecule (e.g., an amplification product). A donor fluorescent moiety, for example, fluorescein, is excited at 470 nm by the light source of the LIGHTCYCLER™ Instrument. During FRET, the fluorescein transfers its energy to an acceptor fluorescent moiety such as LIGHTCYCLER™-Red 640 (LC™-Red 640) or LIGHTCYCLER™-Red 705 (LC™-Red 705). The acceptor fluorescent moiety then emits light of a longer wavelength, which is detected by the optical detection system of the LIGHTCYCLER™ instrument. Efficient FRET can only take place when the fluorescent moieties are in direct local proximity and when the emission spectrum of the donor fluorescent moiety overlaps with the absorption spectrum of the acceptor fluorescent moiety. The intensity of the emitted signal can be correlated with the number of original target nucleic acid molecules (*e.g*., the number of influenza A genomes).

Another FRET format utilizes TAQMAN^{®} technology to detect the presence or absence of an amplification product, and hence, the presence or absence of influenza A. TAQMAN^{®} technology utilizes one single-stranded hybridization probe labeled with two fluorescent moieties. When a first fluorescent moiety is excited with light of a suitable wavelength, the absorbed energy is transferred to a second fluorescent moiety according to the principles of FRET. The second fluorescent moiety is generally a quencher molecule. During the annealing step of the PCR reaction, the labeled hybridization probe binds to the target nucleic acid *(i.e.,* the amplification product) and is degraded by the 5' to 3' exonuclease activity of the Taq Polymerase during the subsequent elongation phase. As a result, the excited fluorescent moiety and the quencher moiety become spatially separated from one another. As a consequence, upon excitation of the first fluorescent moiety in the absence of the quencher, the fluorescence emission from the first fluorescent moiety can be detected. By way of example, an ABI PRISM^{®} 7700 Sequence Detection System (Applied Biosystems, Foster City, CA) uses TAQMAN^{®} technology, and is suitable for performing the methods described herein for detecting influenza A. Information on PCR amplification and detection using an ABI PRISM^{®} 770 system can be found at appliedbiosystems.com/products on the World Wide Web.

Molecular beacons in conjunction with FRET also can be used to detect the presence of an amplification product using the real-time PCR methods described herein Molecular beacon technology uses a hybridization probe labeled with a first fluorescent moiety and a second fluorescent moiety. The second fluorescent moiety is generally a quencher, and the fluorescent labels are typically located at each end of the probe. Molecular beacon technology uses a probe oligonucleotide having sequences that permit secondary structure formation (*e.g*., a hairpin). As a result of secondary structure formation within the probe, both fluorescent moieties are in spatial proximity when the probe is in solution. After hybridization to the target nucleic acids (*i.e*., amplification products), the secondary structure of the probe is disrupted and the fluorescent moieties become separated from one another such that after excitation with light of a suitable wavelength, the emission of the first fluorescent moiety can be detected.

It is understood that the present invention is not limited by the configuration of one or more commercially available instruments.

### Articles of manufacture/Kits

The invention further provides for articles of manufacture to detect influenza A. An article of manufacture according to the present invention, and as defined in the claims, includes primers and probes used to detect influenza A, optionally together with suitable packaging materials. Representative primers and probes for detection of influenza A are capable of hybridizing to influenza A nucleic acid molecules. Methods of designing primers and probes are disclosed herein, and representative examples of primers and probes that amplify and hybridize to influenza A nucleic acid molecules are provided.

Articles of manufacture described herein also can include one or more fluorescent moieties for labeling the probes or, alternatively, the probes supplied with the kit can be labeled. For example, an article of manufacture may include a donor fluorescent moiety for labeling one of the influenza A probes and an acceptor fluorescent moiety for labeling the other influenza A probe, respectively. Examples of suitable FRET donor fluorescent moieties and corresponding acceptor fluorescent moieties are provided above.

Articles of manufacture of the invention also can contain a package insert or package label having instructions thereon for using the influenza A primers and probes to detect influenza A in a sample. Articles of manufacture may additionally include reagents for carrying out the methods disclosed herein (*e.g*., buffers, polymerase enzymes, co-factors, or agents to prevent contamination). Such reagents may be specific for one of the commercially available instruments described herein.

The invention will be further described in the following examples, which do not limit the scope of the invention described in the claims.

### EXAMPLES

### Example 1-Persistence of influenza virus type A in patients

This study included patients ages 18 and older hospitalized with clinical symptoms of influenza virus infections at our institute from December, 2004 through February, 2005. Specimens from patients in the hospital who had influenza-like illness (ILI, as defined by fever, cough, and/or sore throat) were initially tested by real-time PCR for influenza virus type A infection. If the initial real-time PCR assay was positive, subsequent throat swabs were obtained after the original laboratory diagnosis at four time periods (dependent on the length of hospital stay): 48 hours, 72 hours, 5 days, and 7 days. For all testing subsequent to the initial specimen, informed consent was obtained. Patients who were unable to provide serial throat swabs were excluded from the study.

### Example 2-Nucleic acid extraction for RT-PCR

Viral nucleic acid was extracted from throat swabs using either automated or manual extraction. RNA was extracted from 200 µl of patient specimen using the MagNA Pure automated instrument (Roche Applied Science, Indianapolis, IN) and the MagNA Pure Blood/Serum/Plasma extraction program. In addition, nucleic acids were extracted from an Influenza A culture for use as a positive control and from buffer containing 1 x 10³ *E.coli* for use as a negative control. The samples were eluted in 100 µl. As alternative to an automated nucleic acid extraction, RNA was extracted from 200 µl of patient specimen using HighPure (Roche) and eluted with 100 µl of elution buffer.

Five µl of specimen extract was place in 15 µl of master mix and transcribed from RNA to cDNA. Table 1 shows the master mix used for the real-time PCR.

**Table 1. Real-time PCR Master Mix**

| Ingredient | concentration of stock solution | µl of stock solution | final concentration |
|---|---|---|---|
| Water | | 55.7 | |
| Mn ac^{a} | 50 mM | 10 | 2.5 mM |
| Buffer^{b} | 2.7x | 74 | 1x |
| Primer 1 | 50 µM | 1.4 | 0.35 µM |
| Primer 2 | 50 µM | 2.8 | 0.7 µM |
| Probe-FL | 20 µM | 2.0 | 0.2 µM |
| Probe-RED | 20 µM | 4.0 | 0.4 µM |

| | | | |
|---|---|---|---|
| ^{a}, manganese acetate; ^{b}, MagNA Pure buffer or HighPure buffer | | | |

The primer sequences used were: Primer 1: 5'-TAA CCG AGG TCG AAA CGT ATG TTCT-3'; Primer: 2 5'-GGC ATT TTG GAC AAA GCG TCT A-3'; and the probe sequences used were: Probe-FL 5'-CGA AAT CGC GCA GAG ACT TGA AGA TGT-3'; Probe-Red 5'-TTG CTG GGA AAA ACA CAG ATC TTG AGG C-3'.

The product was then amplified and monitored for the development of target nucleic acid sequences after the annealing step during real-time PCR (LIGHTCYCLER®, Roche) cycling using fluorescence resonance energy transfer technology (FRET). Analysis of the real-time PCR amplification and probe melting curves was by LIGHTCYCLER® software. Performance characteristics of the primers and probes used for the detection of influenza A virus RNA were determined by comparing the PCR method described herein with standard cell culture (R-mix cells, Diagnostic Hybrids; see Example 3 below). Table 2 shows the real-time PCR conditions used to amplify and detect influenza A nucleic acid.

**Table 2. Real-time PCR Conditions**

| Program | Cycles | °C | Hold time (mm:ss) | T transition (°C/sec) | Acquisition Mode |
|---|---|---|---|---|---|
| Initial | 1 | 61 | 20:00 | 20 | |
| | | 95 | 3:00 | 20 | |
| PCR | 45 | 95 | 0:10 | 20 | |
| | | 52 | 0:15 | 20 | Single |
| | | 72 | 0:15 | 20 | |
| Melting Curve | 1 | 95 | 0:00 | 20 | |
| | | 55 | 0:20 | 20 | |
| | | 40 | 0:00 | 0.2 | |
| | | 85 | 0:00 | 0.2 | Continuous |
| Cool | 1 | 40 | 0:00 | 20 | |

Of 557 respiratory tract specimens, 49 samples were positive by both PCR and R-mix cell cultures. Forty-three, and two specimens were exclusively positive by PCR and R-mix methods, respectively. Four hundred sixty-three specimens were negative by both methods. This comparison resulted in the following characteristics of the PCR test method compared with standard cell culture: sensitivity, 96%; specificity, 92%; positive predictive value, 53%; and negative predictive value, 100%.

### Example 3-Cell cultures

Throat specimens were extracted into 2 mLs of M5 medium. Aliquots (0.2 mL) were inoculated into each of two shell vial cell cultures containing a cell monolayer of R-Mix (mixed monolayer of human adenocarcinoma cells (A549) and mink lung cells (Mᵥ1Lu) on a 12 mm circular cover slip (Diagnostic Hybrids, Athens, OH). After inoculation, infection of the cells was enhanced by low-speed centrifugation [700 x g (2000 RPM)] for 40 min. After 48 hours of incubation at 35-37°C, monoclonal antibodies (Chemicon International, Inc., Temecula, CA) to influenza virus type A virus were added to coverslips. Viral-specific foci were detected microscopically (100x magnification) after staining with influenza A antisera using direct immunofluorescence.

Extracts of each specimen from study patients also were inoculated in a 0.2 ml volume into primary rhesus monkey kidney (PRMK) tube cell culture and incubated at 35-37 °C for up to 14 days (Diagnostic Hybrids, Athens, OH; and Viromed Laboratories, Minneapolis, MN). Cytopathic effects (CPE) were observed by the formation of large, somewhat irregularly shaped granular cells occurring randomly throughout the culture with cell debris floating in the medium. The presence of influenza A virus in conventional tube cultures was initially detected by hemadsorption using guinea pig erythrocytes and specifically identified by the Binax NOW Influenza A/B kit (Binax Inc., Portland, ME).

### Example 4-Results

Turn-around times were calculated from the time specimens were received into the laboratory to the time the result was reported for real-time PCR and shell vial assays, or completed for tube cell cultures. The average turn-around times for real-time PCR assay were significantly shorter (14.8 hrs) than both the shell vial (49.3 hrs; p<0.001) and tube cell culture (199.2 hrs; p<0.001).

A PCR-positive laboratory test was required for entry of a patient into the study. Of the 50 patients initially positive by PCR, 43 of 50 (86%) were positive by both shell vial and cell culture. Influenza virus type A nucleic acid was detected by PCR in 34 of 50 (68%) of patients at 48 hrs, and in 13 of 41 (31.7%) of patients at 72 hrs after the original positive test. Shell vial and tube cell culture detection were significantly lower at the 48 hr (22/50, 44% and 18/50, 36%, respectively; p<0.001) and 72 hr (6/41, 14.6% and 4/41, 9.8% respectively; p<0.05) time points. Detection of influenza at day 5 was limited to PCR, occurring in (2/8) 25% patients. By day 7, (2/10) 20% patients were positive by PCR; (1/10) 10% of those patients were also detected by shell vial assay. No samples were positive by tube cell culture after day 5.

Influenza A virus infections were detected by sensitive real-time PCR technology in 10 of 50 (20%) study patients after 7 days of hospitalization (Table 3). In contrast, only 5 of 50 (10%) patients were positive by the shell-vial assay after 7 days and none was positive using conventional tube cell culture methods (Table 3). These data suggest that patients can shed influenza A virus beyond the CDC

recommended 5 day droplet isolation period. Importantly, PCR results may not equate with active virus; however, a positive molecular result may indeed indirectly indicate infectious or active virus since cell culture techniques have reduced sensitivity compared to PCR.

**Table 3. Detection of influenza A over 7 days**

| Day | PCR (% detection) | Shell Vial Assay (% detection) | Tube Cell Culture (% detection) |
|---|---|---|---|
| 0 | 100.0 | 86.0 | 86.0 |
| 2 | 68.0 | 44.0 | 36.0 |
| 3 | 31.7 | 14.6 | 9.8 |
| 5 | 25.0 | 0.0 | 0.0 |
| 7 | 20.0 | 10.0 | 0.0 |

## Claims

1. A method for detecting the presence or absence of influenza A in a biological sample from an individual, said method comprising:
performing at least one cycling step, wherein a cycling step comprises an amplifying step and a hybridizing step, wherein said amplifying step comprises contacting said sample with a pair of influenza A primers to produce an influenza A amplification product if an influenza A nucleic acid molecule is present in said sample, wherein said hybridizing step comprises contacting said sample with a pair of influenza A probes, wherein the members of said pair of influenza A probes hybridize within no more than five nucleotides of each other, wherein a first influenza A probe of said pair of influenza A probes is labeled with a donor fluorescent moiety and said second influenza A probe of said pair of influenza A probes is labeled with a corresponding acceptor fluorescent moiety; and
detecting the presence or absence of fluorescence resonance energy transfer (FRET) between said donor fluorescent moiety of said first influenza A probe and said acceptor fluorescent moiety of said second influenza A probe,
wherein the presence of FRET is indicative of the presence of influenza A in said sample, and wherein the absence of FRET is indicative of the absence of influenza A in said sample,
wherein said first influenza A probe comprises the sequence 5'-CGA AAT CGC GCA GAG ACT TGA AGA TGT-3' (SEQ ID NO:3), and wherein said second influenza A probe comprises the sequence 5'-TTG CTG GGA AAA ACA CAG ATC TTG AGG C-3' (SEQ ID NO:4), and
wherein said pair of influenza A primers comprises a first influenza A primer and a second influenza A primer, wherein said first influenza A primer comprises the sequence 5'-TAA CCG AGG TCG AAA CGT ATG TTC T-3' (SEQ ID NO:1), and
wherein said second influenza A primer comprises the sequence 5'-GGC ATT TTG GAC AAA GCG TCT A-3' (SEQ ID NO:2).

2. The method of claim 1, wherein the members of said pair of influenza A probes hybridize within no more than two nucleotides of each other or wherein the members of said pair of influenza A probes hybridize within no more than one nucleotide of each other.

3. The method of claim 1 or 2,
a) wherein said donor fluorescent moiety is fluorescein; or
b) wherein said corresponding acceptor fluorescent moiety is selected from the group consisting of LC-Red 640, LC-Red 705, Cy5, and Cy5.5.

4. The method of any one of claims 1 to 3,
a) wherein said detecting step comprises exciting said sample at a wavelength absorbed by said donor fluorescent moiety and visualizing and/or measuring the wavelength emitted by said corresponding acceptor fluorescent moiety;
b) wherein said detecting comprises quantitating said FRET;
c) wherein said detecting step is performed after each cycling step; or
d) wherein said detecting step is performed in real time.

5. The method of any one of claims 1 to 4,
a) further comprising determining the melting temperature between one or both of said influenza A probe(s) and said influenza A amplification product, wherein said melting temperature confirms said presence or said absence of said influenza A;
b) wherein the presence of said FRET within 45 cycling steps is indicative of the presence of an influenza A infection in said individual;
c) wherein the presence of said FRET within 40 cycling steps is indicative of the presence of an influenza A infection in said individual;
d) wherein the presence of said FRET within 35 cycling steps is indicative of the presence of an influenza A infection in said individual; or
e) further comprising: preventing amplification of a contaminant nucleic acid, wherein said preventing comprises performing said amplifying step in the presence of uracil, especially wherein said preventing further comprises treating said sample with uracil-DNA glycosylase prior to a first amplifying step.

6. The method of claim 5, wherein said preventing comprises performing said amplifying step in the presence of uracil.

7. The method of claim 6, wherein said preventing further comprises treating said sample with uracil-DNA glycosylase prior to a first amplifying step.

8. The method of any one of claims 1 to 7, wherein said biological sample is selected from the group consisting of throat swabs, throat washings, nasal swabs, and specimens from the lower respiratory tract.

9. The method of any one of claims 1 to 8,
a) wherein said cycling step is performed on a control sample, particularly wherein said control sample comprises said portion of said influenza A nucleic acid molecule; or
b) wherein said cycling step uses a pair of control primers and a pair of control probes, wherein said control primers and said control probes are other than said influenza A primers and influenza A probes, wherein said amplifying step produces a control amplification product, wherein said control probes hybridize to said control amplification product.

10. An article of manufacture, comprising:
a pair of influenza A primers, wherein said pair of influenza A primers comprise a first influenza A primer and a second influenza A primer, wherein said first influenza A primer comprises the sequence 5'-TAA CCG AGG TCG AAA CGT ATG TTC T-3' (SEQ ID NO: 1), and wherein said second influenza A primer comprises the sequence 5'-GGC ATT TTG GAC AAA GCG TCT A-3' (SEQ ID NO:2);
a pair of influenza A probes, wherein said pair of influenza A probes comprises a first influenza A probe and a second influenza A probe, wherein said first influenza A probe comprises the sequence 5'-CGA AAT CGC GCA GAG ACT TGA AGA TGT-3' (SEQ ID NO:3), and wherein said second influenza A probe comprises the sequence 5'-TTG CTG GGA AAA ACA CAG ATC TTG AGG C-3' (SEQ ID NO:4), particularly wherein said first influenza A probe is labeled with said donor fluorescent moiety and wherein said second influenza A probe is labeled with said corresponding acceptor fluorescent moiety; and
a donor fluorescent moiety and a corresponding acceptor fluorescent moiety.

11. The article of manufacture of claim 10, further comprising a package insert having instructions thereon for using said pair of influenza A primers and said pair of influenza A probes to detect the presence or absence of influenza A in a sample.

## Patentansprüche

1. Verfahren zum Nachweisen der Gegenwart oder Abwesenheit von Influenza A in einer biologischen Probe aus einem Individuum, wobei das Verfahren umfasst:
Durchführen mindestens eines zyklischen Schrittes, wobei der zyklischer Schritt einen Amplifizierungsschritt und einen Hybridisierungsschritt umfasst, wobei der Amplifizierungsschritt das Inkontaktbringen der Probe mit einem Influenza A-Primerpaar umfasst, um ein Influenza A-Amplifikationsprodukt herzustellen, wenn ein Influenza A-Nukleinsäuremolekül in der Probe vorhanden ist, wobei der Hybridisierungsschritt das Inkontaktbringen der Probe mit einem Influenza A-Sondenpaar umfasst, wobei die Mitglieder des Influenza A-Sondenpaars innerhalb von höchstens 5 Nukleotiden voneinander hybridisieren, wobei eine erste Influenza A-Sonde des Influenza A-Sondenpaars mit einer Donorfluoreszenz-Komponente markiert ist und wobei eine zweite Influenza A-Sonde des Influenza A-Sondenpaars mit einer entsprechenden Akzeptorfluoreszenz-Komponente markiert ist; und
Nachweisen der Gegenwart oder Abwesenheit von Fluoreszenzresonanzenergietransfer (FRET) zwischen der Donorfluoreszenz-Komponente der ersten Influenza A-Sonde und der Akzeptorfluoreszenz-Komponente der zweiten Influenza A-Sonde,
wobei die Gegenwart von FRET für die Gegenwart von Influenza A in der Probe indikativ ist, wobei die Abwesenheit von FRET für die Abwesenheit von Influenza A in der Probe indikativ ist,
wobei die erste Influenza A-Sonde die Sequenz 5'-CGA AAT CGC GCA GAG ACT TGA AGA TGT-3' (SEQ ID NO:3) umfasst und wobei die zweite Influenza A-Sonde die Sequenz 5'-TTG CTG GGA AAA ACA CAG ATC TTG AGG C-3' (SEQ ID NO:4) umfasst, und
wobei das Influenza A-Primerpaar einen ersten Influenza A-Primer und einen zweiten Influenza A-Primer umfasst, wobei der erste Influenza A-Primer die Sequenz 5'-TAA CCG AGG TCG AAA CGT ATG TTC T-3' (SEQ ID NO:1) umfasst und wobei der zweite Influenza A-Primer die Sequenz 5'-GGC ATT TTG GAC AAA GCG TCT A-3' (SEQ ID NO:2) umfasst.

2. Verfahren nach Anspruch 1, wobei die Mitglieder des Influenza A-Sondenpaars innerhalb von höchstens zwei Nukleotiden voneinander hybridisieren oder wobei die Mitglieder des Influenza A-Sondenpaars innerhalb von höchstens einem Nukleotid voneinander hybridisieren.

3. Verfahren nach Anspruch 1 oder 2,
a) wobei die Donorfluoreszenz-Komponente Fluorescein ist; oder
b) wobei die Akzeptorfluoreszenz-Komponente ausgewählt ist aus der Gruppe bestehend aus LC-Red 640, LC-Red 705, Cy5 und Cy5.5.

4. Verfahren nach einem beliebigen der Ansprüche 1 bis 3,
a) wobei der Schritt des Nachweisens das Anregen der Probe bei einer Wellenlänge, die von der Donorfluoreszenz-Komponente absorbiert wird, und Sichtbarmachen und/oder Messen der Wellenlänge, die von der Akzeptorfluoreszenz-Komponente emittiert wird, umfasst;
b) wobei das Nachweisen das Quantifizieren des FRET umfasst;
c) wobei der Schritt des Nachweisens nach jedem zyklischen Schritt durchgeführt wird; oder
d) wobei der Schritt des Nachweisens in Echtzeit durchgeführt wird.

5. Verfahren nach einem beliebigen der Ansprüche 1 bis 4,
a) zusätzlich umfassend das Bestimmen der Schmelztemperatur zwischen einer oder beiden Influenza A-Sonde(n) und dem Influenza A-Amplifikationsprodukt, wobei die Schmelztemperatur die Gegenwart oder Abwesenheit von Influenza A bestätigt;
b) wobei die Gegenwart von FRET innerhalb von 45 Zyklen für die Gegenwart einer Influenza A-Infektion in dem Individuum indikativ ist;
c) wobei die Gegenwart von FRET innerhalb von 40 Zyklen für die Gegenwart einer Influenza A-Infektion in dem Individuum indikativ ist;
d) wobei die Gegenwart von FRET innerhalb von 35 Zyklen für die Gegenwart einer Influenza A-Infektion in dem Individuum indikativ ist; oder
e) weiterhin umfassend das Verhindern der Amplifikation einer kontaminierenden Nukleinsäure, wobei das Verhindern das Durchführen eines Amplifikationsschritts in Gegenwart von Uracil umfasst, insbesondere wobei das Verhindern zusätzlich das Behandeln der Probe mit Uracil-DNA-Glycosylase vor einem ersten Amplifizierungsschritt umfasst.

6. Verfahren nach Anspruch 5, wobei das Verhindern das Durchführen des Amplifikationsschritts in Gegenwart von Uracil umfasst.

7. Verfahren nach Anspruch 6, wobei das Verhindern zusätzlich das Behandeln der Probe mit Uracil-DNA-Glycosylase vor einem ersten Amplifizierungsschritt umfasst.

8. Verfahren nach einem beliebigen der Ansprüche 1 bis 7, wobei die biologische Probe ausgewählt ist aus der Gruppe bestehend aus Rachenabstrichen, Rachenspülungen, Nasenspülungen und Proben aus dem unteren Atmungstrakt.

9. Verfahren nach einem beliebigen der Ansprüche 1 bis 8,
a) wobei der zyklische Schritt mit einer Kontrollprobe durchgeführt wird, insbesondere wobei die Kontrollprobe einen Teil des Influenza A-Nukleinsäuremoleküls umfasst, oder
b) wobei in dem zyklischen Schritt ein Kontrollprimerpaar und ein Kontrollsondenpaar verwendet wird, wobei die Kontrollprimer und die Kontrollsonden sich von den Influenza A -Primern bzw. den Influenza A - Sonden unterscheiden, wobei der Amplifizierungsschritt ein Kontrollamplifikationsprodukt herstellt, wobei die Kontrollsonden mit dem Kontrollamplifikationsprodukt hybridisieren.

10. Artikel aus Herstellung umfassend:
ein Influenza A-Primerpaar, wobei das Influenza A-Primerpaar einen ersten Influenza A-Primer und einen zweiten Influenza A-Primer umfasst, wobei der erste Influenza A-Primer die Sequenz 5'-TAA CCG AGG TCG AAA CGT ATG TTC T-3' (SEQ ID NO:1) umfasst und wobei der zweite Influenza A-Primer die Sequenz 5'-GGC ATT TTG GAC AAA GCG TCT A-3' (SEQ ID NO:2) umfasst;
ein Influenza A-Sondenpaar, wobei das Influenza A-Sondenpaar eine erste Influenza A-Sonde und eine zweite Influenza A-Sonde umfasst, wobei die erste Influenza A-Sonde die Sequenz 5'-CGA AAT CGC GCA GAG ACT TGA AGA TGT-3' (SEQ ID NO:3) umfasst und wobei die zweite Influenza A-Sonde die Sequenz 5'-TTG CTG GGA AAA ACA CAG ATC TTG AGG C-3' (SEQ ID NO:4) umfasst, insbesondere wobei die erste Influenza A-Sonde mit einer Donorfluoreszenz-Komponente markiert ist und wobei eine zweite Influenza A-Sonde mit einer entsprechenden Akzeptorfluoreszenz-Komponente markiert ist; und
eine Donorfluoreszenz-Komponente und eine entsprechende Akzeptorfluoreszenz-Komponente.

11. Artikel aus Herstellung nach Anspruch 10, zusätzlich umfassend einen Beipackzettel mit Instruktionen darauf zur Verwendung des Influenza A-Primerpaars und des Influenza A-Sondenpaars, um die Gegenwart oder Abwesenheit von Influenza A in einer Probe nachzuweisen.

## Revendications

1. Procédé de détection de la présence ou de l'absence d'influenza A dans un échantillon biologique d'un individu, ledit procédé comprenant :
la réalisation d'au moins une étape de cyclage, dans lequel une étape de cyclage comprend une étape d'amplification et une étape d'hybridation, dans lequel ladite étape d'amplification comprend la mise en contact dudit échantillon avec une paire d'amorces d'influenza A pour produire un produit d'amplification d'influenza A si une molécule d'acide nucléique d'influenza A est présente dans ledit échantillon, dans lequel ladite étape d'hybridation comprend la mise en contact dudit échantillon avec une paire de sondes d'influenza A, dans lequel les membres de ladite paire de sondes d'influenza A s'hybrident à pas plus de cinq nucléotides l'une de l'autre, dans lequel une première sonde d'influenza A de ladite paire de sondes d'influenza A est marquée avec une groupement fluorescent donneur et ladite deuxième sonde d'influenza A de ladite paire de sondes d'influenza A est marquée avec un groupement fluorescent accepteur correspondant ; et
la détection de la présence ou de l'absence d'un transfert d'énergie par résonance en fluorescence (FRET) entre ledit groupement fluorescent donneur de ladite première sonde d'influenza A et ledit groupement fluorescent accepteur de ladite deuxième sonde d'influenza A,
dans lequel la présence de FRET est indicative de la présence d'influenza A dans ledit échantillon et dans lequel l'absence de FRET est indicative de l'absence d'influenza A dans ledit échantillon,
dans lequel ladite première sonde d'influenza A comprend la séquence 5'-CGA AAT CGC GCA GAG ACT TGA AGA TGT-3' (SEQ ID NO : 3), et dans lequel ladite deuxième sonde d'influenza A comprend la séquence 5'-TTG CTG GGA AAA ACA CAG ATC TTG AGG C-3' (SEQ ID NO : 4), et
dans lequel ladite paire d'amorces d'influenza A comprend une première amorce d'influenza A et une deuxième amorce d'influenza A, dans lequel ladite première amorce d'influenza A comprend la séquence 5'-TAA CCG AGG TCG AAA CGT ATG TTC T-3'(SEQ ID NO : 1) et dans lequel ladite deuxième amorce d'influenza A comprend la séquence 5'-GGC ATT TTG GAC AAA GCG TCT A-3' (SEQ ID NO : 2).

2. Procédé selon la revendication 1, dans lequel les membres de ladite paire de sondes d'influenza A s'hybrident à pas plus de deux nucléotides l'une de l'autre ou dans lequel les membres de ladite paire de sondes d'influenza A s'hybrident à pas plus d'un nucléotide l'une de l'autre.

3. Procédé selon la revendication 1 ou 2,
a) dans lequel ledit groupement fluorescent donneur est la fluorescéine ; ou
b) dans lequel ledit groupement fluorescent accepteur correspondant est choisi dans le groupe consistant en LC-Red 640, LC-Red 705, Cy5 et Cy5.5.

4. Procédé selon l'une quelconque des revendications **1** à **3**,
a) dans lequel ladite étape de détection comprend l'excitation dudit échantillon à une longueur d'onde absorbée par ledit groupement fluorescent donneur et la visualisation et/ou la mesure de la longueur d'onde émise par ledit groupement fluorescent accepteur correspondant ;
b) dans lequel ladite détection comprend la quantification dudit FRET ;
c) dans lequel ladite étape de détection est réalisée après chaque étape de cyclage ; ou
d) dans lequel ladite étape de détection est réalisée en temps réel.

5. Procédé selon l'une quelconque des revendications **1** à **4**,
a) comprenant en outre la détermination de la température de fusion entre une ou deux desdites sondes d'influenza A, et ledit produit d'amplification d'influenza A, dans lequel ladite température de fusion confirme ladite présence ou ladite absence dudit influenza A ;
b) dans lequel la présence dudit FRET dans 45 étapes de cyclage est indicative de la présence d'une infection à influenza A chez ledit individu ;
c) dans lequel la présence dudit FRET dans 40 étapes de cyclage est indicative de la présence d'une infection à influenza A chez ledit individu ;
d) dans lequel la présence dudit FRET dans 35 étapes de cyclage est indicative de la présence d'une infection à influenza A chez ledit individu ; ou
e) comprenant en outre : la prévention de l'amplification d'un acide nucléique contaminant, dans lequel ladite prévention comprend la réalisation de ladite étape d'amplification en présence d'uracile, en particulier dans lequel ladite prévention comprend en outre le traitement dudit échantillon avec l'uracile-ADN glycosylase avant une première étape d'amplification.

6. Procédé selon la revendication **5**, dans lequel ladite prévention comprend la réalisation de ladite étape d'amplification en présence d'uracile.

7. Procédé selon la revendication **6**, dans lequel ladite prévention comprend en outre le traitement dudit échantillon avec l'uracile-ADN glycosylase avant une première étape d'amplification.

8. Procédé selon l'une quelconque des revendications **1** à **7**, dans lequel ledit échantillon biologique est choisi dans le groupe consistant en des prélèvements de gorge, des lavages de gorge, des prélèvements nasaux et des échantillons prélevés dans les voies respiratoires basses.

9. Procédé selon l'une quelconque des revendications **1** à **8**,
a) dans lequel ladite étape de cyclage est réalisée sur un échantillon témoin, en particulier dans lequel ledit échantillon témoin comprend ladite partie de ladite molécule d'acide nucléique d'influenza A ; ou
b) dans lequel ladite étape de cyclage utilise une paire d'amorces témoins et une paire de sondes témoins, dans lequel lesdites amorces témoins et lesdites sondes témoins sont autres que lesdites amorces d'influenza A et lesdites sondes d'influenza A, dans lequel ladite étape d'amplification produit un produit d'amplification témoin, dans lequel lesdites sondes témoins s'hybrident audit produit d'amplification témoin.

10. Article manufacturé, comprenant :
une paire d'amorces d'influenza A, dans lequel ladite paire d'amorces d'influenza A comprend une première amorce d'influenza A et une deuxième amorce d'influenza A, dans lequel ladite première amorce d'influenza A comprend la séquence 5'-TAA CCG AGG TCG AAA CGT ATG TTC T-3' (SEQ ID NO : 1) et dans lequel ladite deuxième amorce d'influenza A comprend la séquence 5'-GGC ATT TTG GAC AAA GCG TCT A-3' (SEQ ID NO : 2) ;
une paire de sondes d'influenza A, dans lequel ladite paire de sondes d'influenza A comprend une première sonde d'influenza A et une deuxième sonde d'influenza A, dans lequel ladite première sonde d'influenza A comprend la séquence 5'-CGA AAT CGC GCA GAG ACT TGA AGA TGT-3' (SEQ ID NO: 3) et dans lequel ladite deuxième sonde d'influenza A comprend la séquence 5'-TTG CTG GGA AAA ACA CAG ATC TTG AGG C-3' (SEQ ID NO : 4), en particulier dans lequel ladite première sonde d'influenza A est marquée avec ledit groupement fluorescent donneur et dans lequel ladite deuxième sonde d'influenza A est marquée avec ledit groupement fluorescent accepteur correspondant ; et
un groupement fluorescent donneur et un groupement fluorescent accepteur correspondant.

11. Article manufacturé selon la revendication 10, comprenant en outre une notice d'emballage comportant des instructions sur celle-ci pour l'utilisation de ladite paire d'amorces d'influenza A et de ladite paire de sondes d'influenza A pour détecter la présence ou l'absence d'influenza A dans un échantillon.
